# EUROPEAN PATENT SPECIFICATION

(11) **EP 1 656 910 B1**
(45) Date of publication and mention of the grant of the patent: **03.09.2008**
(21) Application number: 05110476.8
(22) Date of filing: 08.11.2005
(51) Int. Cl.: A61F 2/40

(54) **Inverse prosthesis for the articulation of the shoulder**
Inverse Prothese für das Schultergelenk
Prothèse inversée pour l'articulation de l'épaule

(30) Priority: 10.11.2004 IT UD20040208
(43) Date of publication of application: 17.05.2006
(73) Proprietor: LIMA Lto SpA, 33030 Villanova di San Daniele del Friuli (IT)
(72) Inventor: Dalla Pria, Paolo, 33100, UDINE (IT)
(74) Representative: Petraz, Davide Luigi

(56) References cited:
- GB-A- 1 362 187
- US-A- 3 916 451
- US-A1- 2004 220 673

## Description

### FIELD OF THE APPLICATION

The present invention concerns a prosthesis of the shoulder for the articulation of the head of a humerus with respect to the relative scapula. To be more exact, the prosthesis according to the present invention is configured as an inverse prosthesis, wherein a convex-shaped head is associated with the glenoid cavity and articulates in an artificial concave seating associated with the top of the humerus.

### BACKGROUND OF THE INVENTION

An inverse prosthesis of the shoulder is known, comprising a spherical element attached to the scapula which articulates in a mating cavity located in the upper part of the humerus. This prosthesis is called "inverse" because it reproduces in an inverse manner the glenohumeral anatomy.

Normally, the inverse prosthesis is used instead of normal prostheses in cases of serious muscular degeneration of the shoulder, particularly of the muscles of the rotator cuff.

The inverse prosthesis normally comprises an attachment rod or pin, inserted into the humerus, which allows to attach and position, usually by means of a supporting body, a humeral cup or insert.

The spherical element articulates in the humeral cup, and is in turn attached to the glenoid cavity. The spherical element is in turn normally provided with a pin for direct attachment in the glenoid cavity, or to a support previously attached to the glenoid cavity.

Solutions that are normally used provide that the humeral cup is made of plastic material, for example polyethylene, and that the spherical element is made of metal, in order to guarantee good characteristics of sliding and low wear to the artificial articulation.

In order to guarantee the correct functioning of the inverse prosthesis and to prevent mechanical yielding of the part of the prosthesis that is constrained to the glenoid cavity, it is generally provided that the center of the articulation is displaced towards the latter. In this way the outer edge of the humeral cup is often in contact with the bony part of the scapula, causing it to rub against the latter.

As a consequence of this rubbing, an effect of mechanical scraping of the bony part of the scapula is generated; moreover, the plastic material deteriorates and can release particles that cause an effect of osteolysis and a consequent considerable re-absorption of the bone. This bone re-absorption can lead progressively to a thinning of the bony part on which the prosthesis is anchored, compromising the mechanical stability of the part of the prosthesis constrained to the glenoid cavity, and determining a limited duration of the correct functioning of the inverse prosthesis applied.

US-A-3.916.451 **discloses an inverse prosthesis comprising a ceramic spherical head, which articulates on a humeral cup made of metal or ceramic material.**

GB-A-l.362.187 **discloses an inverse prosthesis comprising a spherical head which articulates in a humeral cup. Both the spherical head and the humeral cup may be made either of plastic material or of metal material.**

One purpose of the invention is to achieve an inverse prosthesis for the articulation of the shoulder which eliminates, simply and without having recourse to additional components, the problem of rubbing of the plastic part of the prosthesis on the bony part, keeping the characteristics of good sliding and low wear deriving from the association of an anti-friction plastic material and a metal material with good characteristics of anchorage to the bony part.

The Applicant has devised, tested and embodied the present invention to overcome the shortcomings of the state of the art and to obtain these and other purposes and advantages.

### SUMMARY OF THE INVENTION

The present invention is set forth and characterized in the main claim, while the dependent claims describe other characteristics of the invention or variants to the main inventive idea.

In accordance with the above purpose, an inverse prosthesis of the shoulder is used for the articulation of a humerus in a scapula of a shoulder having a glenoid cavity. The prosthesis according to the present invention comprises a first articulation element, at least partly concave and associated with the top of the humerus, and a second articulation element, partly convex and associated with the glenoid cavity.

According to a characteristic of the present invention, **the second articulation element comprises an outer part and an interposition element.**

At least the outer part of the second articulation element or glenoid element, which articulates on the first articulation element or humeral element, is made of or lined with plastic or ceramic material; the first articulation element, associated with the humerus, is made of a material, such as metal, metal alloy or even ceramic material, particularly non-polymeric material, which does not release particles and which therefore does not cause the phenomenon of osteolysis, even if it is subjected to rubbing against the bony part of the scapula.

The second, convex articulation element is associated with attachment means at least partly metal which anchors it to the glenoid cavity.

In this way, as we said, it is possible to achieve the humeral element of anti-wear material, inverting the choice of the materials of conventional inverse prostheses, and thus eliminating the problem of rubbing of plastic parts on the bony part and the consequent unwanted release of particles.

For example, the humeral element can be made of metal, metal alloy with a titanium, cobalt or other base, or of ceramic material or other analogous or comparable material, provided it is not polymeric and hence not subject to the above-mentioned phenomenon of releasing particles when subjected to rubbing against the bony part of the scapula.

According a preferred embodiment of the invention, the attachment means that anchors the glenoid element to the glenoid cavity advantageously comprises at least a metal attachment pin and said interposition element, made of metal, which creates the connection between the metal pin and the outer part, or articulation part, of the glenoid element made of plastic or ceramic material.

The interposition element consists, in a preferential embodiment, of an annular bush, bushing or washer which is housed and is fixed in a suitable inner seating provided in the outer part, plastic or ceramic, of the glenoid element, and allows coupling to the pin without risks of yielding, wear or weakness of the attachment.

The metal interposition element can be clamped onto the outer part of the glenoid element by simple interference, screwing, pins, nails, screws, gluing or any other known manner.

As we said, the use of a glenoid element made in at least two parts of different material, an inner metal attachment part and an outer part made of plastic or ceramic material, allows on the one hand to guarantee stability and durability of the attachment to the scapula, and on the other hand allows to use humeral elements made of metal or ceramic material, and in any case not polymeric, reducing and even eliminating the problems of rubbing against the bony part of the scapula and the consequent release of particles.

### BRIEF DESCRIPTION OF THE DRAWINGS

These and other characteristics of the present invention will become apparent from the following description of a preferential form of embodiment, given as a non-restrictive example with reference to the attached drawings wherein:
- fig. 1 is a longitudinal section of an inverse prosthesis according to the present invention implanted in a shoulder;
- fig. 2 is an exploded view of a detail of fig. 1;
- fig. 3 is an exploded view of a variant of a detail in fig. 1.

### DETAILED DESCRIPTION OF A PREFERENTIAL FORM OF EMBODIMENT

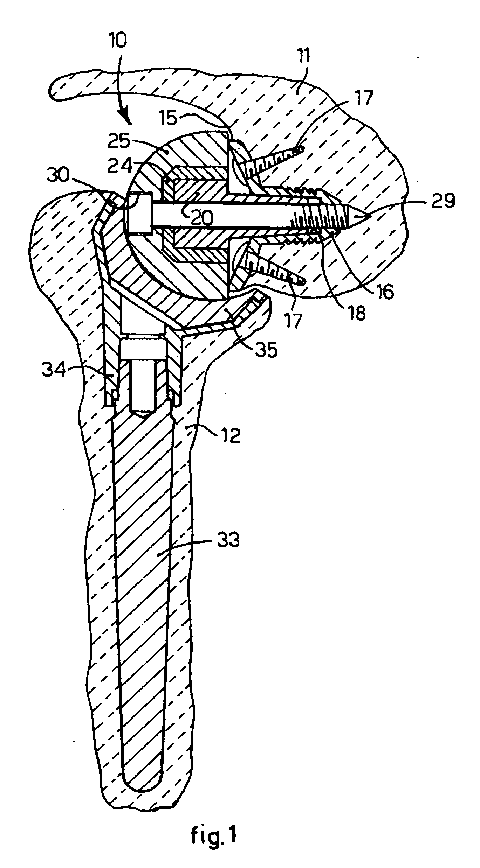

With reference to fig. 1, an inverse prosthesis 10 according to the present invention is implanted in a shoulder, to allow the articulation of a humerus 12 in a relative glenoid cavity 15 of a scapula 11.

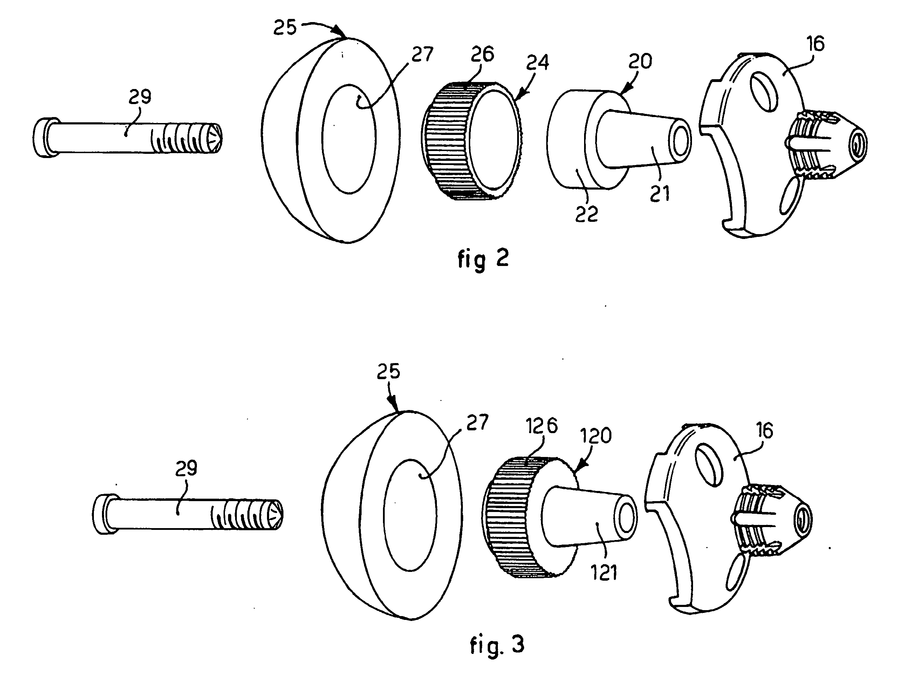

To be more exact, the inverse prosthesis 10 comprises, on the side of the scapula 11, a metal support 16 (figs. 1 and 2), normally called glenoid, inserted for example under pressure in a seating made in the glenoid cavity 15 and attached to the latter by means of screws 17.

The support 16 has an axial cavity 18 inside which, in the solution shown here, a conical front part 21 of a metal pin 20 is inserted.

According to a variant solution of the present invention, not shown, it is provided to attach the pin 20 to the support 16 by screwing, or it is also provided to make the pin 20 and the support 16 in a single piece.

The pin 20 has a rear part 22 substantially cylindrical in shape on which an annular metal bushing 24 is inserted, which in the case shown here has a lined or knurled outer surface 26.

According to a variant not shown here, the outer surface of the annular bushing 24 has fins, or other types of interference means on the surface, so as to achieve a coupling in shape and/or by force, as described hereafter.

A glenoid head 25, also called glenosphere, preferably made of plastic material, for example polyethylene or similar, is assembled on the annular bushing 24, in this case through simple interference; it has a hemispherical outer shape and comprises inside a seating 27 mating with the outer surface 26, in this case knurled, of the annular bushing 24.

During the assembly step of the inverse prosthesis 10, which normally but not necessarily is performed before it is implanted into the patient, the glenoid head 25 is coupled with the annular bushing 24, inserting with force the outer surface 26 of the latter into the seating 27, so as to achieve substantially a single element with the outer part made of anti-friction plastic material and the inner part of metal material.

The annular bushing 24 thus performs the function of an interposition element between the convex outer body of the glenoid head 25 which functions as an articulation part proper, and the inner body which cooperates in anchoring it to the scapula 11.

According to a variant solution, not shown here, the outer surface 26 of the annular bushing 24 has a threading and the seating 27 has a mating threading which cooperate in order to attach through screwing the glenoid head 25 to the annular bushing 24.

According to another variant solution not shown here, the annular bushing 24 and the glenoid head 25 are attached reciprocally by means of at least a pin, a screw or other, inserted for example into a corresponding aperture made both on the annular bushing 24 and also on the pin 20.

According to another variant solution not shown here, the outer surface 26 and the seating 27 have a conical shape so as to allow a reciprocal conical coupling and increase the interference and stability over time between the glenoid head 25 and the annular bushing 24.

Furthermore, every other type of attachment of the annular bushing 24 and the glenoid head 25, for example by gluing, can be used and comes within the field of the present invention.

In the case shown, the pin 20, the annular bushing 24 and the glenoid head 25 also have a longitudinal through hole so as to accommodate an attachment screw 29, advantageously used as a safety element, in order to anchor the whole thus obtained to the glenoid cavity 15.

In this case, on the outer surface, the glenoid head 25 has a hollow 30 in which the head of the attachment screw 29 is invisibly housed during use.

According to a variant not shown here, the support or glenoid 16 couples with the bushing 24 by forced insertion of the respective peripheral edge into an appropriate seating made on the perimeter of the bushing 24 itself.

The inverse prosthesis 10 also comprises, on the side of the humerus 12, an attachment rod 33 inserted into the humerus 12, which allows to attach and position, by means of a supporting body 34, a concave humeral cup 35 preferably made of metal or ceramic material, in which the convex surface of the glenoid head 25 articulates.

The use of a humeral cup 35 of metal or ceramic material, and in any case not plastic and/or polymeric, as on the contrary normally happens in state-of-the-art inverse prostheses, as we said, allows to reduce and even eliminate the problems deriving from the rubbing of plastic parts against the bony part of the scapula 11 and hence the unwanted release of particles that cause osteolysis.

The use of interposition elements, such as the pin 20 and the annular bushing 24, made of metal material, allows to anchor the glenoid head 25, made of plastic or ceramic material, to the scapula 11, without risks of yielding, or weakness in attachment.

Therefore, thanks to the inversion of the materials used with respect to conventional inverse prostheses of the scapula, we obtain the advantage of guaranteeing a great stability over time for the assembled prosthesis, preventing thinning and yielding of the bony attachment part, and guaranteeing that a long time will pass before possible revisions or replacements of the prosthesis need to be made.

It is clear that modifications and/or additions of parts may be made to the inverse prosthesis 10 as described heretofore, without departing from the field and scope of the present invention.

According to a variant which is not part of the present invention, instead of the pin 20 and the annular bushing 24, it is provided to use a pin 120 (fig. 3) which has in a single piece a conical front portion 121 inserted during use into the axial cavity 18 and a cylindrical outer surface 126 for coupling with the seating 27 of the glenoid head 25.

In the case shown here the outer surface 126 is lined, but it is possible to provide all the variants described for the outer surface 26 of the annular bushing 24, for example fins, ridges, teeth, or anything else suitable to achieve a coupling of form and/or by force with the inner surface of the seating 27 of the spherical glenoid head 25.

It is also clear that, although the present invention has been described with reference to some specific examples, a person of skill in the art shall certainly be able to achieve many other equivalent forms of inverse prosthesis for the articulation of the shoulder, having the characteristics as set forth in the claims and hence all coming within the field of protection defined thereby.

## Claims

1. Inverse prosthesis of the shoulder for the articulation of a humerus (12) in a scapula (11) of a shoulder having a glenoid cavity (15), comprising a first articulation element (35), at least partly concave and attached to the top of said humerus (12), and a second articulation element (25), partly convex, associated with said glenoid cavity (15) by means of metal attachment means (16, 17, 20, 29, 120), wherein said second articulation element (25) comprises An outer part and an interposition element (24), wherein said outer part of said second articulation element (25), is made of or lined by plastic or ceramic material, in that said first articulation element (35) is made of non-polymeric, anti-wear, metal or ceramic material, **characterised in that** said interposition element, (24) is an annular element made of metal and located and rigidly blocked inside said outer part of second articulation element (25) in order to achieve substantially a single element with said articulation element (25) and to create a connection between said outer part of the second articulation element (25) and said metal attachment means (16, 17, 20, 29).

2. Inverse prosthesis as in claim 1, **characterized in that** said first articulation element (35) is made of a metal alloy based on titanium, cobalt or other.

3. Inverse prosthesis as in claim 1, **characterized in that** said attachment means comprises, at least a supporting element (16, 17) able to be anchored to said glenoid cavity (15), and at least a pip-type attachment element (20) able to connect said outer part of said second articulation element (25) to said supporting element (16, 17).

4. Inverse prosthesis as in claim 1, **characterized in that** said annular element (24) is a bush, bushing or washer able to be coupled with a mating seating (27) made inside said outer part of said second articulation element (25).

5. Inverse prosthesis as in claim 4, **characterized in that** said annular element (24) includes a knurled or lined or finned outer surface, able to be inserted and clamped through interference in said seating (27).

6. Inverse prosthesis as in claim 4, **characterized in that** said annular element (24) is able to be screwed into said seating (27).

7. Inverse prosthesis as in claim 4, **characterized in that** said annular element (24) is able to be clamped in said seating (27) by means of pins, screws, glue or other.

8. Inverse prosthesis as in claims 1 and 3, **characterized in that** said annular element (24) and said metal pin-type element (20) are made in separate pieces and are connected each other by means of a connection part (22).

9. Inverse prosthesis as in claims 1 and 3, **characterized in that** said metal pin-type element (20) is at least partly conical.

10. Inverse prosthesis as in claim 1, **characterized in that** said outer part of said second articulation element (25) is made of polyethylene.

## Patentansprüche

1. Inversprothese für die Schulter zur gelenkigen Verbindung eines Oberarmknochens (12) mit einem Schulterblatt (11) einer eine Schultergelenkpfanne (15) aufweisenden Schulter mit einem ersten Gelenkelement (35), das im Wesentlichen konkav und an dem Kopf des Oberarmknochens (12) angebracht ist, und mit einem zweiten Gelenkelement (25), das im Wesentlichen konvex und mittels eines metallischen Befestigungsmittels (16, 17, 20, 29, 120) mit der Schultergelenkpfanne (15) verbunden ist, wobei das zweite Gelenkelement (25) ein äußeres Teil und ein Zwischenelement (24) aufweist, wobei das äußere Element des zweiten Gelenkelementes (25) aus einem Kunststoff- oder Keramikmaterial hergestellt oder mit diesem überzogen ist, wobei das erste Gelenkelement (35) aus einem nicht polymerhaltigen, verschleißfesten Metall- oder Keramikmaterial hergestellt ist, **dadurch gekennzeichnet, dass** das Zwischenelement (24) ein ringförmiges Element ist, das aus Metall hergestellt und innerhalb des äußeren Teils des zweiten Gelenkelementes (25) angeordnet sowie starr verblockt ist, um mit diesem Gelenkelement (25) eine im Wesentlichen bauliche Einheit zu bilden und eine Verbindung zwischen dem äußeren Teil des zweiten Gelenkelementes (25) sowie dem metallischen Befestigungsmittel (16, 17, 20, 29) herzustellen.

2. Inversprothese nach Anspruch 1, **dadurch gekennzeichnet, dass** das erste Gelenkelement (35) aus einer Metalllegierung auf der Basis von Titan, Kobalt oder dergleichen hergestellt ist.

3. Inversprothese nach Anspruch 1, **dadurch gekennzeichnet, dass** das Befestigungsmittel wenigstens ein Trägerelement (16, 17) umfasst, das dazu eingerichtet ist, in der Schultergelenkpfanne (15) verankert zu werden, und über wenigstens ein stiftartiges Befestigungselement (20) verfügt, das dazu eingerichtet ist, das äußere Teil des zweiten Gelenkelementes (25) mit dem Trägerelement (16, 17) zu verbinden.

4. Inversprothese nach Anspruch 1, **dadurch gekennzeichnet, dass** das ringartige Element (24) eine Buchse, eine Hülse oder eine Ringscheibe ist, die dazu eingerichtet ist, mit einer zugehörigen Aufnahme (27) verbunden zu werden, die innerhalb des äußeren Teils des zweiten Gelenkelementes (25) ausgebildet ist.

5. Inversprothese nach Anspruch 4, **dadurch gekennzeichnet, dass** das ringförmige Element (24) über eine gerändelte oder unebene oder gerippte äußere Oberfläche verfügt, die dazu eingerichtet ist, mittels Übermaß in die Aufnahme (27) eingefügt und verklemmt zu werden.

6. Inversprothese nach Anspruch 4, **dadurch gekennzeichnet, dass** das ringförmige Element (24) dazu eingerichtet ist, in die Aufnahme (27) eingeschraubt zu werden.

7. Inversprothese nach Anspruch 4, **dadurch gekennzeichnet, dass** das ringförmige Element (24) dazu eingerichtet ist, mittels Stiften, Schrauben, Klebstoff oder dergleichen mit der Aufnahme (27) verbunden zu werden.

8. Inversprothese nach Anspruch 1 und 3, **dadurch gekennzeichnet, dass** das ringförmige Element (24) und das metallische stiftartige Element (20) als verschiedene Teile hergestellt und mittels eines Verbindungsteils (22) miteinander verbunden sind.

9. Inversprothese nach Anspruch 1 und 3, **dadurch gekennzeichnet, dass** das metallische stiftartige Element (20) wenigstens teilweise konisch ausgebildet ist.

10. Inversprothese nach Anspruch 1, **dadurch gekennzeichnet, dass** das zweite Gelenkelement (25) aus Polyethylen hergestellt ist.

## Revendications

1. Prothèse inversée de l'épaule pour l'articulation d'un humérus (12) dans une scapula (54) d'épaule ayant une cavité glénoïde (15), comportant un premier élément articulaire (35) au moins partiellement concave et fixé en haut dudit humérus (12), et un second élément articulaire (25) partiellement convexe, associé à ladite cavité glénoïde (15) par des moyens de fixation métalliques (16, 17, 20, 29, 120), dans laquelle ledit second élément articulaire (25) comprend une partie externe et un élément intercalaire (24), ladite partie externe dudit second élément articulaire (25) étant constituée ou doublée de matériau plastique ou céramique, ledit premier élément articulaire (35) étant constitué de matériau non polymère, anti-usure, métallique ou céramique, prothèse **caractérisée en ce que** ledit élément intercalaire (24) est un élément annulaire en métal, placé et bloqué de façon rigide à l'intérieur de ladite partie externe du second élément articulaire (25) afin de former sensiblement un seul élément avec ledit élément articulaire (25) et de créer un assemblage entre ladite partie externe du second élément articulaire (25) et lesdits moyens de fixation métalliques (16, 17, 20, 29).

2. Prothèse inversée selon la revendication 1, **caractérisée en ce que** ledit premier élément articulaire (35) est constitué d'un alliage métallique à base de titane, de cobalt ou autre.

3. Prothèse inversée selon la revendication 1, **caractérisée en ce que** lesdits moyens de fixation comprennent au moins un élément de support (16, 17) pouvant être ancré à ladite cavité glénoïde (15), et au moins un élément de fixation de type broche pouvant assembler ladite partie externe dudit second élément articulaire (25) audit élément de support (16, 17).

4. Prothèse inversée selon la revendication 1, **caractérisée en ce que** ledit élément annulaire (24) est une bague, douille ou rondelle pouvant être accouplée à un siège apparié (27) ménagé à l'intérieur de ladite partie externe dudit second élément articulaire (25).

5. Prothèse inversée selon la revendication 5, **caractérisée en ce que** ledit élément annulaire (24) comprend une surface externe moletée, striée ou à ailettes, pouvant être introduite et bloquée par interaction dans ledit siège (27).

6. Prothèse inversée selon la revendication 5, **caractérisée en ce que** ledit élément annulaire (24) peut être vissé dans ledit siège (27).

7. Prothèse inversée selon la revendication 5, **caractérisée en ce que** ledit élément annulaire (24) peut être bloqué dans ledit siège (27) au moyen de broches, vis, colle ou autre.

8. Prothèse inversée selon les revendications 1 et 3, **caractérisée en ce que** ledit élément annulaire (24) et ledit élément métallique de type broche (20) sont fabriqués dans des pièces distinctes et sont assemblés entre eux par une pièce d'assemblage (22).

9. Prothèse inversée selon les revendications 1 et 3, **caractérisée en ce que** ledit élément métallique de type broche (20) est au moins partiellement conique.

10. Prothèse inversée selon la revendication 1, **caractérisée en ce que** ladite partie externe dudit second élément articulaire (25) est en polyéthylène.
